(19) Europäisches Patentamt / European Patent Office / Office européen des brevets

(11) **EP 2 858 717 B1**

(12) **FASCICULE DE BREVET EUROPEEN**

(45) Date de publication et mention
de la délivrance du brevet:
**30.11.2016 Bulletin 2016/48**

(21) Numéro de dépôt: **13744810.6**

(22) Date de dépôt: **07.06.2013**

(51) Int Cl.:
*A61N 1/378* (2006.01)     *A61B 5/00* (2006.01)
*A61N 1/372* (2006.01)

(86) Numéro de dépôt international:
**PCT/IB2013/054685**

(87) Numéro de publication internationale:
**WO 2013/186678 (19.12.2013 Gazette 2013/51)**

(54) **ALIMENTATION PHOTOVOLTAIQUE TRANSCUTANEE D'UN DISPOSITIF ELECTRONIQUE OU ELECTRIQUE IMPLANTE**

TRANSKUTANE PHOTOVOLTAISCHE VERSORGUNG EINER IMPLANTIERTEN ELEKTRONISCHEN ODER ELEKTRISCHEN VORRICHTUNG

TRANSCUTANEOUS PHOTOVOLTAIC SUPPLY OF AN IMPLANTED ELECTRONIC OR ELECTRICAL DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **11.06.2012 FR 1255456**

(43) Date de publication de la demande:
**15.04.2015 Bulletin 2015/16**

(73) Titulaire: **Commissariat à l'Énergie Atomique et aux Énergies Alternatives 75015 Paris (FR)**

(72) Inventeurs:
- **KARST, Nicolas
  57600 Folkling (FR)**
- **PERRAUD, Simon
  83150 Bandol (FR)**

(74) Mandataire: **Gevers & Orès
41 avenue de Friedland
75008 Paris (FR)**

(56) Documents cités:
**WO-A2-02/30264          US-A- 6 091 015
US-A1- 2006 085 051**

- **MURAKAWA K ET AL: "A WIRELESS NEAR-INFRARED ENERGY SYSTEM FOR MEDICAL IMPLANTS A LESS INVASIVE METHOD FOR SUPPLYING LIGHT POWER TO IMPLANT DEVICES", IEEE ENGINEERING IN MEDICINE AND BIOLOGY MAGAZINE, IEEE SERVICE CENTER, PISACATAWAY, NJ, US, vol. 18, no. 6, 1 novembre 1999 (1999-11-01), pages 70-72, XP000870869, ISSN: 0739-5175, DOI: 10.1109/51.805148 cité dans la demande**

**Description**

La présente description porte sur un système constitué par un dispositif électronique ou électrique implanté - notamment de type électromédical - comprenant un convertisseur photovoltaïque, et par un dispositif permettant de l'alimenter ou le recharger de manière non-intrusive, par éclairage transcutané dudit convertisseur.

L'invention est définie dans les revendications qui suivent. On constate ces dernières années un intérêt croissant pour les dispositifs médicaux sans fil implantables, tels que les stimulateurs cardiaques, les stimulateurs neuronaux, et les capteurs permettant une détection et un contrôle biomédical *in vivo*. Ces systèmes pourront permettre par exemple un diagnostic précoce de certaines maladies ou encore de contrôler précisément et en temps réel le taux de sucre ou d'oxygène présent dans le sang. Cependant, afin de fonctionner de façon autonome, ces objets nécessitent une alimentation interne, généralement assurée par une batterie ou un supercondensateur. Les batteries, dont les densités de puissance sont relativement élevées (jusqu'à 300Wh/litre), permettent d'alimenter des petits systèmes sur une durée allant de quelques jours à quelques années en fonction de la puissance nécessaire. Ainsi, pour les systèmes devant rester implantés pendant une longue période (plusieurs années, voire toute la vie du patient) un renouvellement ou une recharge régulière des batteries s'impose. Or, pour les applications in vivo, le remplacement des batteries n'est pas anodin et nécessite une intervention chirurgicale plus ou moins lourde en fonction du lieu d'implantation de l'objet. Pour cette raison, de nombreuses équipes à travers le monde ont cherché à développer des méthodes non intrusives pour transférer de l'énergie de l'extérieur vers l'intérieur d'un corps humain ou animal de manière à alimenter directement ou indirectement (en rechargeant une batterie) ces systèmes implantés.

Le document US 6,400,991 décrit un système utilisant plusieurs bobines primaires externes afin de transférer, par induction, de l'énergie électromagnétique à une bobine secondaire implantée. Un dispositif de contrôle permet d'identifier la ou les bobines primaires qui sont les plus proches de la bobine secondaire en analysant le décalage de leur fréquence de résonance, puis de diriger de manière sélective le courant électrique à travers ces bobines primaires. Cependant, l'alimentation des bobines primaires nécessite une énergie importante, difficilement compatible avec un système portable. De plus, le transfert d'énergie par induction électromagnétique peut poser des problèmes d'échauffement par effet Joule du dispositif implanté, ainsi que des problèmes d'interférences électromagnétiques.

Le document US 8,082,041 décrit un système de transmission d'énergie basé sur un transducteur piézoélectrique externe, générant des ultrasons, et un récepteur piézoélectrique implanté, convertissant ces ultrasons en énergie électrique. Un tel système ne peut fonctionner de manière efficace que si le transducteur et le récepteur sont alignés précisément. Pour cette raison, quatre capteurs d'ultrasons sont disposés sur le périmètre du récepteur implanté, agencés en carré. Ces capteurs captent l'onde acoustique transmise par le transducteur externe ; lorsque le signal reçu par les capteurs est équilibré, l'alignement du transducteur et du récepteur est optimal. Ce système présente l'inconvénient d'être relativement complexe ; en outre, la procédure d'alignement peut être longue et fastidieuse.

L'article de K. Murakawa et al. « A wireless near-infrared energy system for medical implants », IEEE Engineering Medecine Biology 18, 70 (1999) propose d'utiliser un laser émettant dans le proche infrarouge, couplé à un convertisseur ou générateur photovoltaïque pour alimenter une batterie. Le convertisseur photovoltaïque est implanté sous la peau d'un patient et éclairé par le laser, situé à l'extérieur du corps du patient, lorsque la batterie doit être rechargée. Cependant, afin que la conversion du rayonnement soit réellement efficace il est nécessaire que le laser soit parfaitement aligné avec le convertisseur. Cela est d'autant plus important que, pour atteindre des tensions suffisamment élevées pour alimenter un appareil électromédical (de l'ordre de 3 V), le convertisseur doit le plus souvent comprendre plusieurs cellules photovoltaïques élémentaires connectées en série (typiquement, de 5 à 10). Or, dans ces conditions il suffit qu'une seule cellule soit mal éclairée du fait d'un alignement imparfait avec le laser pour que la puissance électrique générée par le convertisseur photovoltaïque chute fortement.

Pour résoudre ce problème, le document US 6,091,015 propose d'amener la lumière au convertisseur photovoltaïque par l'intermédiaire d'une fibre optique biocompatible implantée. Cependant, une telle solution ne peut pas être qualifiée de « non intrusive » ; en pratique, le fait d'amener de la lumière à l'intérieur du corps par l'intermédiaire d'une fibre optique n'apporte pas d'avantages comparé au fait d'amener directement de l'électricité par l'intermédiaire de fils biocompatibles.

Le document US 2006/0085051 divulgue un module externe monté à même le corps transmettant de l'énergie dans le spectre lumineux à travers la peau à un module interne qui convertit ladite énergie en courant continu via un film récepteur photoélectrique. Le courant continu peut être utilisé pour charger sans effraction des batteries alimentant un implant ou pour alimenter directement un implant. Ledit document divulgue l'ensemble des caractéristiques du préambule de la revendication 1.

L'invention vise à surmonter les inconvénients précités de l'art antérieur. Plus précisément, l'invention vise à permettre l'alimentation directe ou indirecte (c'est-à-dire par l'intermédiaire d'une batterie, qui est rechargée) d'un dispositif électronique ou électrique implanté par une méthode non intrusive, simple à mettre en oeuvre, ne nécessitant pas de procédures d'alignement fastidieuses et, de préférence, utilisant un appareil d'alimentation externe portable.

Un objet de l'invention permettant d'atteindre ce but est un système comprenant :

- un dispositif électronique ou électrique, implantable dans un corps humain ou animal, comprenant un convertisseur photovoltaïque pour assurer son alimentation en énergie électrique ; et
- un dispositif d'éclairage, destiné à être disposé à l'extérieur dudit corps humain ou animal, pour effectuer un éclairage transcutané dudit convertisseur photovoltaïque ;

système dans lequel
ledit dispositif électronique ou électrique implantable comprend :

- un instrument de mesure de la puissance électrique générée par ledit convertisseur photovoltaïque ; et
- un émetteur, pour transmettre un signal représentatif de ladite puissance électrique ;

et dans lequel
ledit dispositif d'éclairage comprend :

- une pluralité de sources de lumière pouvant être allumées ou éteintes individuellement ;
- un récepteur, pour recevoir ledit signal représentatif de la puissance électrique générée par ledit convertisseur photovoltaïque ; et
- un contrôleur pour calculer, à partir dudit signal, une efficacité de transfert énergétique entre ledit dispositif d'éclairage et ledit convertisseur photovoltaïque, et pour modifier la répartition spatiale de l'éclairage provenant desdites sources de lumière de manière à maximiser ladite efficacité ou assurer qu'elle soit supérieure à un seuil prédéterminé.

Selon différents modes de réalisation particuliers de l'invention :

- Ledit contrôleur peut être également configuré ou programmé pour ajuster la puissance lumineuse émise par chaque source de lumière allumée de manière à maximiser ladite efficacité de transfert énergétique ou assurer qu'elle soit supérieure à un seuil prédéterminé.
- Ledit émetteur peut être constitué par ledit convertisseur photovoltaïque.
- Ledit émetteur peut comprendre des moyens pour moduler un rayonnement lumineux incident sur ledit dispositif électronique ou électrique implantable et le rediriger vers ledit dispositif d'éclairage.
- La surface sur laquelle sont effectivement agencées lesdites sources de lumière peut présenter une aire supérieure à celle de la surface photosensible dudit convertisseur photovoltaïque.
- Lesdites sources de lumière peuvent présenter une émission lumineuse multidirectionnelle.
- Ledit dispositif d'éclairage peut comprendre un film transparent recouvrant lesdites sources de lumière, l'indice optique dudit film étant choisi de manière à réaliser une adaptation d'indice avec la peau dudit corps humain ou animal. En outre, ledit film transparent peut être recouvert d'une couche de gel transparent, l'indice optique dudit gel étant choisi de manière à réaliser une adaptation d'indice avec la peau dudit corps humain ou animal.

- Ledit dispositif d'éclairage peut comprendre un support souple et conformable (« un patch »). Avantageusement, la face dudit support souple et conformable portant lesdites sources de lumière peut être recouverte d'une couche de colle transparente permettant sa fixation à la peau dudit corps humain ou animal, l'indice optique de ladite couche de colle étant choisi de manière à réaliser une adaptation d'indice avec ladite peau.
- Ledit support peut être transparent ou semi-transparent sur une partie au moins de sa surface.
- Ladite ou chaque dite source de lumière peut être semi-transparente.

Ladite ou chaque dite source de lumière peut être une diode électroluminescente.

- Ledit dispositif d'éclairage peut être adapté pour émettre un rayonnement lumineux dans la plage spectrale allant de 300 à 1900 nm et de préférence dans la plage spectrale allant de 750 à 1200 nm.
- Ledit dispositif électronique ou électrique implantable peut comprendre un élément de stockage de l'énergie électrique générée par ledit convertisseur photovoltaïque.
- Ledit convertisseur photovoltaïque peut être constitué d'une pluralité de cellules photovoltaïque interconnectées électriquement en série.
- Le dispositif d'éclairage peut comprendre un modulateur de l'intensité lumineuse émise par lesdites sources de lumière, pour transmettre de l'information audit dispositif électronique ou électrique implantable, et ledit dispositif électronique ou électrique implantable peut comprendre un récepteur pour relever des variations du courant ou de la tension générée par ledit convertisseur photovoltaïque et en extraire ladite information.

D'autres caractéristiques, détails et avantages de l'invention ressortiront à la lecture de la description faite en référence aux dessins annexés donnés à titre d'exemple et qui représentent, respectivement :

- La figure 1, un dispositif d'éclairage selon un mode de réalisation de l'invention ;
- La figure 2, une vue d'ensemble d'un système selon un mode de réalisation de l'invention ;
- La figure 3, un graphique illustrant la dépendance de la transmission optique de la peau porcine en fonction de la longueur d'onde ;

- La figure 4, un schéma fonctionnel du fonctionnement d'un système selon un mode de réalisation de l'invention ;
- La figure 5, l'utilisation de repères tatoués sur la peau du patient pour réaliser un alignement approximatif entre le dispositif d'éclairage et le convertisseur photovoltaïque implanté dans un système selon un mode de réalisation de l'invention ; et
- La figure 6, un dispositif d'éclairage selon un autre mode de réalisation de l'invention.

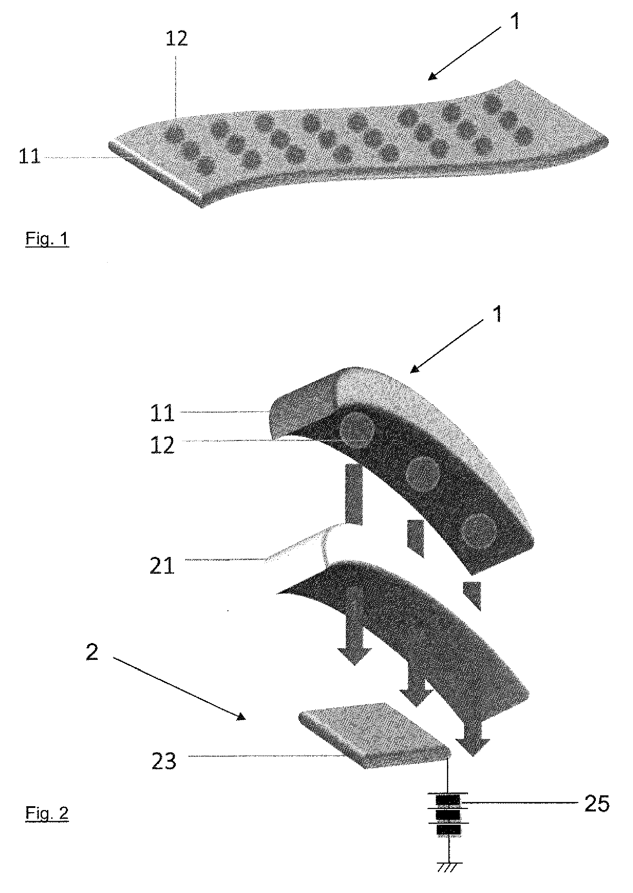

La figure 1 montre un dispositif d'éclairage 1 constitué par un support 11 souple dont l'une des faces (ou surfaces principales) porte une pluralité de diodes électroluminescentes (DEL) 12. Les DEL sont alimentées par une batterie, située sur la face opposée de l'élément, ou par une alimentation externe par l'intermédiaire de fils électriques traversant l'élément et sortant de cette même face opposée.

Le support 11 constitue un patch souple, réalisé en un matériau flexible tel qu'un polymère (par exemple : polyéthylène, polypropylène, polyéthylène téréphtalate, polytétrafluoroéthylène), voire un métal (par exemple : aluminium, titane, acier) à condition que son épaisseur soit suffisamment faible. Ce patch est conformable et peut être adapté à la forme de la région du corps humain ou animal à l'intérieur duquel est implanté le dispositif à alimenter.

La face de l'élément 11 portant les DEL 12 - ou plus précisément la portion de cette face sur laquelle les DEL sont effectivement agencées (définie comme la plus petite région convexe contenant toutes les sources) - présente de préférence une aire supérieure (de préférence, supérieur d'au moins 10% et jusqu'à un facteur 10) à celle du convertisseur photovoltaïque implanté ; cela permet d'obtenir un éclairage satisfaisant du convertisseur photovoltaïque même si les surfaces émettrice et photosensible ne sont pas parfaitement alignées. Par exemple, un convertisseur implanté dans l'avant-bras d'un patient peut présenter une surface photosensible d'aire comprise entre $1 \text{ mm}^2$ et $50 \text{ cm}^2$, et la surface émettrice de lumière de l'élément 11 peut alors présenter une aire comprise entre $1 \text{ cm}^2$ et $100 \text{ cm}^2$. Les surfaces photoémettrices des différentes DEL 12 peuvent être séparées par une distance s. Dans ce cas, il est préférable que $s$ soit inférieur ou de l'ordre de $2d \tan \alpha$, où d est la distance entre la surface émettrice de lumière et la surface photosensible du convertisseur photovoltaïque (qui dépend notamment de la profondeur d'implantation de ce dernier), et où $\alpha$ est l'angle pour lequel l'intensité lumineuse émise par une DEL est divisée par 2 par rapport à la normale au support 11.

Toujours dans le but d'obtenir un éclairage satisfaisant du convertisseur photovoltaïque même lorsque l'alignement est approximatif, il est avantageux que les sources de lumière présentent une émission lumineuse multidirectionnelle, de préférence avec $10° \leq \alpha \leq 30°$. Cette condition est généralement satisfaite par des DEL.

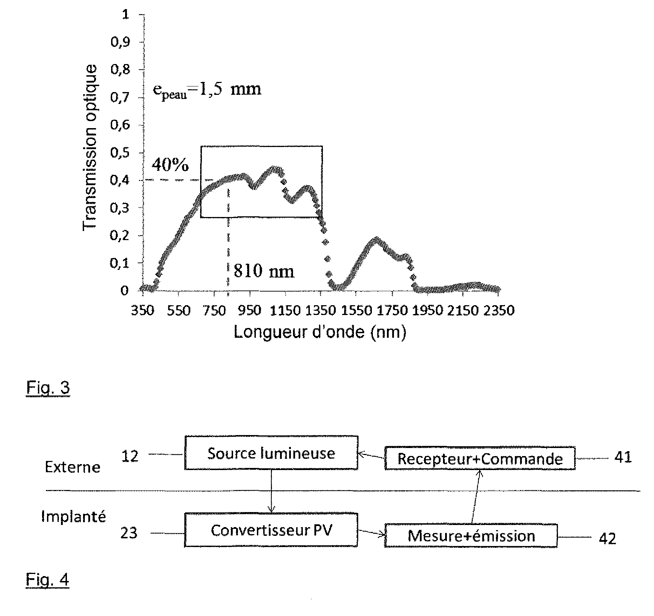

De préférence, les DEL 12 émettent dans le proche infrarouge (750 - 1200 nm) du fait de la grande transparence des tissus biologiques à ces longueurs d'onde (voir la figure 3: transmission de la lumière à travers d'une peau de porc de 1,5 mm d'épaisseur). Il peut s'agir par exemple de DEL à base d'arséniure de gallium (GaAs). Plus généralement, l'émission peut se situer dans une plage spectrale allant du proche infrarouge au proche ultraviolet (300 - 1900 nm).

La figure 2 montre une vue d'ensemble d'un système selon un mode de réalisation de l'invention. Sur cette figure, la référence 2 indique le dispositif électronique ou électrique implanté dans sa globalité. L'élément 21 (qui ne fait pas partie du système) correspond à un tissu biologique - typiquement la peau - qui est traversé par le rayonnement lumineux généré par les DEL 12 portées par le patch 11. L'épaisseur de ce tissu biologique dépend de l'implantation du convertisseur photovoltaïque, entre quelques micromètres et quelques centimètres. L'élément 23 correspond au convertisseur photovoltaïque implanté sous le tissu biologique 21, faisant partie du dispositif 2. Le convertisseur photovoltaïque 23 peut par exemple être basé sur des matériaux absorbeurs en couches minces, tels que le $Cu(In_{16x},Ga_x)(Se_{1-y},S_y)_2$ (CIGS), le $Cu_2ZnSn(Se_{1-y},S_y)_4$ (CZTS), le silicium amorphe, ou le silicium microcristallin ; ces couches minces sont déposées sur un substrat tel qu'une plaque de verre, une feuille d'acier ou de titane, ou encore une feuille de polyimide. Le convertisseur photovoltaïque 23 peut être également basé sur des matériaux absorbeurs massifs, tels que le silicium, l'arséniure de gallium (GaAs) ou le phosphure d'indium (InP). Le choix du matériau absorbeur dépend en partie de la gamme spectrale d'émission des DEL 12. Plus précisément, le rendement quantique externe du convertisseur photovoltaïque 23 doit être élevé (de préférence supérieur à 50%) dans la gamme spectrale d'émission des DEL 12. Par exemple, si les DEL 12 sont des DEL à base d'arséniure de gallium émettant vers 850 - 950 nm, alors un matériau absorbeur particulièrement performant est le $CuInSe_2$ ou le $cu(In_{0.7},Ga_{0.3})Se_2$. Le convertisseur photovoltaïque 23 (ainsi que le reste du dispositif 2 dont il assure l'alimentation) est encapsulé par une couche biocompatible et transparente aux longueurs d'ondes utilisées pour l'éclairage, par exemple en parylène. Pour une application biomédicale (capteur de glucose, par exemple) consommant une puissance d'environ 10 μW, on peut utiliser un convertisseur photovoltaïque composé de 6 cellules photovoltaïques connectées en série, présentant une surface photosensible d'environ $1 \text{ cm}^2$ et produisant une tension de 3 V et un courant de 5 μA sous un éclairage reçu par le convertisseur photovoltaïque d'environ $1 \text{ mW/cm}^2$. Une micro-batterie 25 d'une tension de 3 V, faisant également partie du dispositif 2, peut jouer le rôle de tampon entre le capteur biomédical et le convertisseur photovoltaïque, permettant ainsi de ne pas utiliser les DEL en permanence, mais seulement pour charger la micro-batterie (« alimentation indirecte »). Pour une micro-batterie d'une capacité de 5 mW·h chargée pendant 30 minutes à l'aide du convertisseur photovoltaïque fournissant 10 mW, un capteur consommant 10 μW peut avoir une autonomie de 500 heures. Ainsi le patient est dans l'obligation de recharger son implant toutes les 3 semaines environ.

Les DEL 12 peuvent être allumées ou éteintes individuellement, et leur puissance d'émission lumineuse être réglée de façon indépendante. Ainsi, il est possible de sélectionner un sous-ensemble de DEL à allumer et ajuster leur luminosité pour maximiser l'efficacité de transfert énergétique, c'est-à-dire le rapport η entre la puissance électrique générée par le convertisseur photovoltaïque et la puissance électrique alimentant les DEL. Ainsi, l'optimisation de l'efficacité de transfert énergétique est obtenue en modifiant la répartition spatiale de l'éclairage provenant des sources de lumière. Avantageusement, cette optimisation peut être conduite de manière automatique à l'aide d'une boucle de contrôle illustrée schématiquement sur la figure 4. Sur cette figure, le bloc 42 correspond à un ensemble constitué par un instrument de mesure de la puissance électrique générée par ledit convertisseur photovoltaïque (par exemple, un circuit électronique de mesure de la tension et du courant), intégré au dispositif implanté et par un émetteur (par exemple radiofréquence ou infrarouge) transmettant un signal - analogique ou, de préférence, numérique - représentatif de la puissance électrique mesurée. Côté dispositif d'éclairage, le bloc 41 comprend un récepteur pour ledit signal et un contrôleur (par exemple, un microprocesseur programmé de manière opportune) recevant en entrée le signal et pilotant l'allumage et l'alimentation des DEL de manière à maximiser le rapport η - ou à assurer que ce rapport prenne une valeur supérieure ou égale à un seuil prédéfini.

Dans le cas où l'émetteur 42 est un émetteur infrarouge, plusieurs modes de réalisation peuvent être envisagés.

Dans un premier mode de réalisation, l'émetteur 42 peut être constitué d'une diode électroluminescente, par exemple à base d'arséniure de gallium.

Dans un deuxième mode de réalisation, l'émetteur 42 peut être constitué par le convertisseur photovoltaïque lui-même ; en effet, une cellule photovoltaïque et une diode électroluminescente présentent la même architecture de base, à savoir une jonction entre un matériau semi-conducteur de type p et un matériau semi-conducteur de type n. Par exemple, il est connu que les cellules photovoltaïques à base de CIGS sont capables d'émettre de la lumière dans le proche infrarouge lorsqu'elles sont excitées électriquement.

Dans un troisième mode de réalisation, présentant l'avantage de réduire la consommation d'énergie du dispositif implanté 2, l'émetteur 42 peut être constitué par un système effectuant les opérations suivantes :

1) récupérer une partie du flux lumineux infrarouge émis par les DEL 12, et incident sur le dispositif 2 ;

2) moduler l'amplitude de ce flux lumineux de manière à y coder le signal à transmettre ;

3) réémettre ce flux lumineux en direction du récepteur 41.

Un tel système peut être constitué d'un modulateur électro-optique à guide d'onde (par exemple à base de silicium, arséniure de gallium, ou LiNbO$_3$) et de deux réseaux de diffraction définis sur la même surface que le guide d'onde. Le premier réseau de diffraction permet d'injecter une partie du flux lumineux incident dans le guide d'onde ; le modulateur électro-optique à guide d'onde permet ensuite de moduler le flux lumineux circulant dans le guide d'onde ; le deuxième réseau de diffraction permet enfin d'extraire le flux lumineux du guide d'onde pour le réémettre en direction du récepteur 41.

Un exemple de procédure de communication entre l'émetteur implanté 42 et le bloc de réception et de commande 41 du dispositif d'éclairage est le suivant.

Tout d'abord, le patch doit être aligné de manière approximative avec le convertisseur photovoltaïque (pré alignement) de façon à ce qu'un minimum de puissance lumineuse puisse parvenir jusqu'au convertisseur photovoltaïque. Une fois cette étape réalisée, la procédure débute avec toutes les DEL allumées. Le contrôleur détermine la valeur initiale du rapport η, valeur devant être supérieure à une valeur préenregistrée témoignant d'un pré alignement acceptable. Si la valeur de η déterminée par le contrôleur est inférieure à une valeur préenregistrée un signal sonore pourra être émis par le bloc de réception et de commande 41 afin d'informer le patient que l'étape de pré alignement doit être renouvelée. Si la valeur de η déterminée par le contrôleur est supérieure à une valeur préenregistrée ce dernier commande l'extinction d'une première DEL située par exemple à une extrémité. Après l'extinction de la première DEL il détermine à nouveau le rapport η afin de vérifier si celui-ci est différent de la valeur initiale. Si celui-ci est strictement inférieur à la valeur initiale ou à une valeur préenregistrée, alors le contrôleur commande de rallumer cette DEL. Si au contraire celui-ci est supérieur ou égal à la valeur initiale ou à une valeur préenregistrée, alors le contrôleur laisse la première DEL éteinte et commande l'extinction d'une deuxième DEL, par exemple voisine de la première. Le même contrôle que précédemment est effectué conduisant à l'extinction ou au rallumage de la seconde DEL en fonction de la valeur du rapport mesurée dans cette configuration. Cette procédure est appliquée jusqu'à ce que toutes les DEL aient été testées. Une autre possibilité consisterait à tester l'ensemble des combinaisons possibles, c'est-à-dire l'ensemble des combinaisons de k DEL pris parmi *n (n* étant le nombre de DEL et *k* un nombre entier variant de 1 à n ; le nombre de combinaisons possible est égal à

$$\sum_{k=1}^{k=n} C_n^k \text{ avec } C_n^k = \frac{n!}{k!(n-k)!}),$$ et de garder la combinaison qui maximise le rapport η. Il est à noter que ce rapport η dépend de l'épaisseur de tissus biologiques traversée et du rendement de conversion du convertisseur photovoltaïque. Ainsi pour une épaisseur de tissu traversée de 1,5 mm et pour un rendement de conversion du convertisseur photovoltaïque de 20 % on peut estimer que ce rapport η devra être supérieur ou égal à 12%. Grâce à ce système, il n'est pas nécessaire d'aligner précisément le patch avec le convertisseur photovoltaïque pour maximiser le rapport η.

Après avoir pré aligné le patch et après avoir effectué la procédure de maximisation du rapport η par une répartition spatiale appropriée de l'éclairage provenant des DEL, l'éclairage transcutané du convertisseur photovoltaïque peut débuter. Par la suite, une vérification périodique de la valeur du rapport η peut être réalisée afin de s'assurer que la valeur du rapport η reste toujours égale à la valeur maximale. Si tel n'est pas le cas (par exemple du fait d'un glissement accidentel du patch), alors

- un signal sonore peut être émis par le bloc de réception et de commande 41 afin d'informer le patient que l'étape de pré alignement doit être de nouveau effectuée,
- ou bien la procédure de maximisation du rapport η peut être à nouveau effectuée.

Dans le cas de l'exemple présenté sur la figure 2, deux DEL sont nécessaires pour maximiser le rapport η, tandis que la troisième DEL peut être éteinte pour réaliser une économie d'énergie tout en assurant une charge optimale de la micro-batterie.

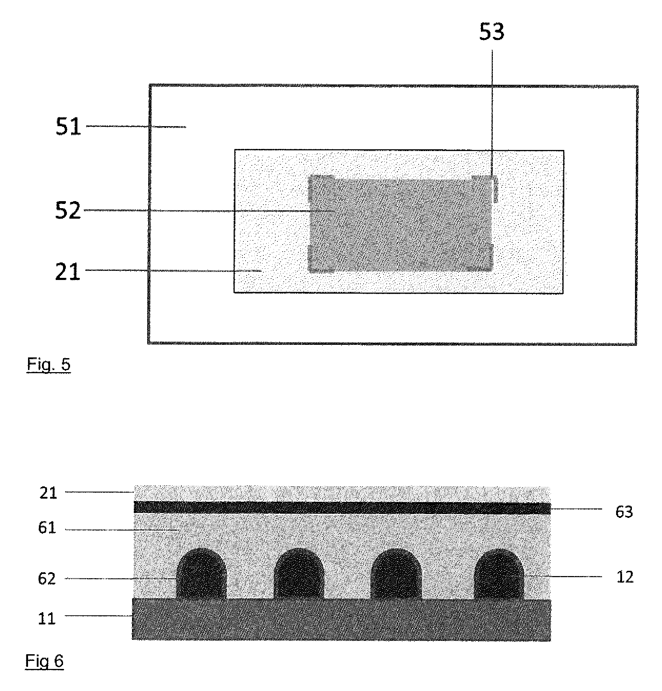

Selon un mode de réalisation avantageux, illustré par la figure 5, le patch 51 peut être transparent ou semi-transparent, en tout ou en partie, en dehors des sources de lumière. Cela permet de réaliser un alignement approximatif des sources de lumière avec le convertisseur photovoltaïque, dont les contours peuvent être matérialisés sur la peau par l'intermédiaire d'un tatouage 53. Pour faciliter l'alignement, les sources lumineuses peuvent aussi être semi-transparentes ; par exemple, il est connu de réaliser des DEL semi-transparentes dans lesquelles l'électrode en face arrière est réalisée en un oxyde transparent conducteur tel que $SnO_2$ ou ITO (oxyde d'indium et étain) ou est constitué par une ou plusieurs couches métalliques (Ag, Al, etc.) très fines (5 - 20nm).

Comme illustré à la figure 6, un film 61 peut recouvrir ou encapsuler les DEL 12 et permettre ainsi de planariser la face avant du dispositif d'éclairage pour assurer un meilleur contact avec la peau et mieux épouser la forme du corps. Le film 61, d'épaisseur typiquement comprise entre 1 $\mu$m et 5 mm, est préférentiellement transparent (transmission optique>80%) dans la gamme spectrale d'émission des DEL 12. Le film 61 est préférentiellement constitué d'un matériau polymère souple et transparent, par exemple : polyéthylène, polypropylène, polyméthacrylate de méthyle, cellulose, polycarbonate, polyéthylène téréphtalate, parylène. Le film 61, outre d'assurer un bon contact avec la peau, permet de disposer d'une chaîne d'indices optiques favorable afin de limiter la réflexion à la surface de la peau de la lumière émise par les DEL 12. En effet, en l'absence du film 61, la lumière émise par les DEL 12 devra traverser une couche d'air avant d'atteindre la peau. Or la différence d'indice optique entre l'air (indice d'environ 1) et la peau (indice typiquement compris dans la gamme 1.3 - 1.5) est importante, ce qui entraîne une réflexion importante de la lumière et donc une baisse de l'efficacité de transfert énergétique. Au contraire, en présence du film 61, la lumière émise par les DEL 12 traverse une couche de polymère avant d'atteindre la peau. Or la différence d'indice optique entre un polymère (indice typiquement compris dans la gamme 1,4 - 1,6) et la peau (indice typiquement compris dans la gamme 1,3 - 1,5) est faible, ce qui limite les phénomènes de réflexion optique et limite donc la baisse de l'efficacité de transfert énergétique (adaptation d'indice). Il faut noter que les DEL 12 comprennent généralement une encapsulation 62 constituée d'une résine époxy. Le film 61 peut recouvrir cette encapsulation 62, ou bien se substituer à cette encapsulation 62.

Afin d'améliorer le contact du film 61 avec la peau, et d'éliminer le risque d'une formation d'une couche d'air entre le film 61 et la peau, un gel (et notamment un hydrogel) ou un adhésif 63 peut être placé entre le film 61 et la peau. L'hydrogel est constitué d'eau et d'un polymère réticulé (par exemple à base de pectine, alginate de calcium ou carboxymethylcellulose de sodium). L'adhésif peut être préférentiellement un adhésif transparent et hypoallergénique, par exemple à base de polyacrylates. Son utilisation présente l'avantage additionnel de faciliter le maintien en place du support en forme de patch 11.

Le dispositif d'éclairage peut également être utilisé pour transmettre au dispositif implanté des signaux véhiculant une information, par exemple pour configurer, programmer ou piloter ledit dispositif implanté. Pour ce faire, ledit dispositif d'éclairage peut comprendre un modulateur de l'intensité lumineuse émise par lesdites sources de lumière, l'information à transmettre étant codée par des variations de ladite intensité lumineuse. Ces variations provoquent à leur tour des variations dans la tension ou le courant généré par le convertisseur photovoltaïque, qui peuvent aisément être détectées par un récepteur afin d'extraire l'information ainsi transmise.

L'invention a été décrite en référence à un certain nombre de modes de réalisation particuliers, mais plusieurs variantes sont envisageables.

Ainsi, par exemple, l'utilisation de DEL (organiques ou inorganiques) en tant que sources de lumière est particulièrement préférée en raison de leur faible coût, de leur consommation réduite et de leur émission spatialement incohérente, et donc multidirectionnelle, qui contribue à un éclairage uniforme du générateur photovoltaïque même lorsque ce dernier n'est pas parfaitement aligné avec le patch 11. Cependant, d'autres sources de lumière telles que des diodes laser peuvent être envisagées.

De même, il n'est pas essentiel que ces sources émettent dans le proche infrarouge. En variante, il serait possible d'utiliser des sources de lumière visible, par exemple blanche, même si la transmission transcutanée serait réduite.

Le dispositif électronique ou électrique implanté ne doit pas nécessairement comprendre un capteur. Il pourrait par exemple s'agir d'un stimulateur neuronal, voire même d'un émetteur radio permettant de suivre les déplacements d'un animal non-humain.

La présence d'une batterie ou super-condensateur n'est pas non plus essentielle : en variante, on peut envisager une alimentation directe du dispositif par le convertisseur photovoltaïque. Dans ce cas, le dispositif fonctionne uniquement pendant l'éclairage, ce qui peut être suffisant pour certaines applications.

Le support des sources de lumière ne doit pas nécessairement se présenter sous la forme d'un patch souple, même si un tel mode de réalisation est particulièrement avantageux.

Le contrôleur pilotant l'allumage et l'extinction des sources de lumière peut être porté par le support ou bien être déporté, en communication filaire ou sans fils avec le récepteur.

L'invention est définie par les revendications suivantes:

## Revendications

**1.** Système comprenant :

- un dispositif électronique ou électrique (2), implantable dans un corps humain ou animal, comprenant un convertisseur photovoltaïque (23) pour assurer son alimentation en énergie électrique ; et
- un dispositif d'éclairage (1), destiné à être disposé à l'extérieur dudit corps humain ou animal, pour effectuer un éclairage transcutané dudit convertisseur photovoltaïque ;

système dans lequel: ledit dispositif électronique ou électrique implantable comprend :

- un instrument de mesure (42) de la puissance électrique générée par ledit convertisseur photovoltaïque ; et
- un émetteur (42), pour transmettre un signal représentatif de ladite puissance électrique ;

et dans lequel
ledit dispositif d'éclairage comprend :

- un récepteur (41), pour recevoir ledit signal représentatif de la puissance électrique générée par ledit convertisseur photovoltaïque ; et

**caractérisé en ce que** le dispositif d'éclairage comprend en outre:

- une pluralité de sources de lumière pouvant être allumées ou eteintes individuellement; et
- un contrôleur (41) pour calculer, à partir dudit signal, une efficacité de transfert énergétique entre ledit dispositif d'éclairage et ledit convertisseur photovoltaïque, et pour allumer ou éteindre individuellement lesdites sources de lumière de manière à maximiser ladite efficacité ou assurer qu'elle soit supérieure à un seuil prédéterminé.

**2.** Système selon la revendication 1, dans lequel ledit contrôleur est également configuré ou programmé pour ajuster la puissance lumineuse émise par chaque source de lumière allumée de manière à maximiser ladite efficacité de transfert énergétique ou assurer qu'elle soit supérieure à un seuil prédéterminé.

**3.** Système selon l'une des revendications précédentes, dans lequel ledit émetteur (42) est constitué par ledit convertisseur photovoltaïque.

**4.** Système selon l'une des revendications 1 ou 2 dans lequel ledit émetteur (42) comprend des moyens pour moduler un rayonnement lumineux incident sur ledit dispositif électronique ou électrique implantable et le rediriger vers ledit dispositif d'éclairage.

**5.** Système selon l'une des revendications précédentes, dans lequel la surface sur laquelle sont effectivement agencées lesdites sources de lumière présente une aire supérieure à celle de la surface photosensible dudit convertisseur photovoltaïque.

**6.** Système selon l'une des revendications précédentes, dans lequel lesdites sources de lumière présentent une émission lumineuse multidirectionnelle.

**7.** Système selon l'une des revendications précédentes dans lequel ledit dispositif d'éclairage comprend un film transparent (61) recouvrant lesdites sources de lumière, l'indice optique dudit film étant choisi de manière à réaliser une adaptation d'indice avec la peau dudit corps humain ou animal.

**8.** Système selon la revendication 7 dans lequel ledit film transparent est recouvert d'une couche de gel transparent, l'indice optique dudit gel étant choisi de manière à réaliser une adaptation d'indice avec la peau dudit corps humain ou animal.

**9.** Système selon l'une des revendications précédentes, dans lequel ledit dispositif d'éclairage comprend un support (11) souple et conformable.

**10.** Système selon la revendication 9, dans lequel la face dudit support (11) souple et conformable portant lesdites sources de lumière est recouverte d'une couche de colle transparente permettant sa fixation à la peau dudit corps humain ou animal, l'indice optique de ladite couche de colle étant choisi de manière à réaliser une adaptation d'indice avec ladite peau.

**11.** Système selon l'une des revendications précédentes, dans lequel ledit support est transparent ou semi-transparent sur une partie au moins de sa surface.

**12.** Système selon la revendication 11, dans lequel ladite ou chaque dite source de lumière est semi-transparente.

**13.** Système selon l'une des revendications précédentes, dans lequel ledit dispositif d'éclairage est adapté pour émettre un rayonnement lumineux dans la plage spectrale allant de 300 à 1900 nm et de préférence dans la plage spectrale allant de 750 à 1200 nm.

**14.** Système selon l'une des revendications précédentes, dans lequel ledit dispositif électronique ou électrique implantable comprend un élément de stockage (25) de l'énergie électrique générée par ledit convertisseur photovoltaïque.

**15.** Système selon l'une des revendications précédentes, dans lequel le dispositif d'éclairage comprend un modulateur de l'intensité lumineuse émise par lesdites sources de lumière, pour transmettre de l'information audit dispositif électronique ou électrique implantable, et ledit dispositif électronique ou électrique implantable comprend un récepteur pour relever des variations du courant ou de la tension générée par ledit convertisseur photovoltaïque et en extraire ladite information.

**Patentansprüche**

**1.** System, umfassend:

- eine in den Körper eines Menschen oder Tiers implantierbare elektrische oder elektronische Vorrichtung (2), umfassend einen photovoltaischen Wandler (23) zur Sicherung ihrer Versorgung mit elektrischer Energie, und
- eine Beleuchtungsvorrichtung (1), die bestimmt ist, außerhalb des Körpers des Menschen oder des Tiers angeordnet zu sein, um eine transkutane Beleuchtung des photovoltaischen Wandlers durchzuführen,

wobei bei dem System:

die implantierbare elektrische oder elektronische Vorrichtung umfasst:

- ein Messinstrument (42) der von dem photovoltaischen Wandler erzeugten elektrischen Leistung, und
- einen Sender (42), um ein für die elektrische Leistung repräsentatives Signal zu übertragen,

und wobei die Beleuchtungsvorrichtung umfasst:

- einen Empfänger (41), um das für die von dem photovoltaischen Wandler erzeugte elektrische Leistung repräsentative Signal zu empfangen, und

**dadurch gekennzeichnet, dass** die Beleuchtungsvorrichtung ferner umfasst:

- eine Vielzahl von Lichtquellen, die individuell ein- oder ausschaltbar sind, und
- einen Controller (41), um auf der Basis des Signals eine energetische Transfereffizienz zwischen der Beleuchtungsvorrichtung und dem photovoltaischen Wandler zu berechnen und um die Lichtquellen individuell ein- oder auszuschalten, um die Effizienz zu maximieren oder um sicherzustellen, dass sie über einem vorbestimmten Grenzwert liegt.

**2.** System nach Anspruch 1, wobei der Controller ebenfalls konfiguriert oder programmiert ist, um die von jeder eingeschalteten Lichtquelle gesendete Lichtleistung derart anzupassen, dass die energetische Transfereffizienz maximiert wird oder um sicherzustellen, dass sie über einem vorbestimmten Grenzwert liegt.

**3.** System nach einem der vorangehenden Ansprüche, wobei der Sender (42) von dem photovoltaischen Wandler gebildet ist.

**4.** System nach einem der Ansprüche 1 oder 2, wobei der Sender (42) Mittel umfasst, um eine auf die implantierbare elektrische oder elektronische Vorrichtung einfallende Lichtstrahlung zu modulieren und um sie zu der Beleuchtungsvorrichtung zurückzuschicken.

**5.** System nach einem der vorangehenden Ansprüche, wobei die Oberfläche, auf welcher die Lichtquellen tatsächlich ausgebildet sind, eine Fläche aufweist, die höher ist als die der photosensiblen Oberfläche des photovoltaischen Wandlers.

**6.** System nach einem der vorangehenden Ansprüche, wobei die Lichtquellen eine multidirektionale Lichtsendung aufweisen.

7. System nach einem der vorangehenden Ansprüche wobei die Beleuchtungsvorrichtung eine transparente Folie (61) aufweist, die die Lichtquellen bedeckt, wobei der optische Index der Folie derart gewählt ist, dass eine Indexanpassung an die Haut des Körpers des Menschen oder Tiers stattfindet.

8. System nach Anspruch 7, wobei die transparente Folie mit einer transparenten Gelschicht bedeckt ist, wobei der optische Index des Gels derart gewählt ist, dass eine Indexanpassung an die Haut des Körpers des Menschen oder Tiers stattfindet.

9. System nach einem der vorangehenden Ansprüche, wobei die Beleuchtungsvorrichtung eine elastische und komfortable Unterlage (11) umfasst.

10. System nach Anspruch 9, wobei die Seite der elastischen und komfortablen Unterlage (11), welche die Lichtquellen trägt, mit einer transparenten Kleberschicht bedeckt ist, die ihre Befestigung auf der Haut des Körpers des Menschen oder Tiers erlaubt, wobei die Kleberschicht derart gewählt ist, dass eine Indexanpassung an die Haut stattfindet.

11. System nach einem der vorangehenden Ansprüche, wobei die Unterlage über mindestens einen Teil ihrer Oberfläche transparent oder halbtransparent ist.

12. System nach Anspruch 11, wobei die oder jede Lichtquelle halbtransparent ist.

13. System nach einem der vorangehenden Ansprüche, wobei die Beleuchtungsvorrichtung ausgebildet ist, um eine Lichtstrahlung im Spektralbereich von 300 bis 1900 nm und vorzugsweise im Spektralbereich von 750 bis 1200 nm zu senden.

14. System nach einem der vorangehenden Ansprüche, wobei die implantierbare elektrische oder elektronische Vorrichtung ein Speicherelement (25) der von dem photovoltaischen Wandler erzeugten elektrischen Energie umfasst.

15. System nach einem der vorangehenden Ansprüche, wobei die Beleuchtungsvorrichtung einen Modulator der von den Lichtquellen gesendeten Lichtstärke umfasst, um Informationen an die implantierbare elektrische oder elektronische Vorrichtung zu übertragen, und die implantierbare elektrische oder elektronische Vorrichtung einen Empfänger umfasst, um die von dem photovoltaischen Wandler erzeugten Strom- oder Spannungsschwankungen zu erfassen und die Information daraus zu entnehmen.

## Claims

1. A system comprising:

- an electronic or electrical device (2), implantable in a human or animal body, comprising a photovoltaic converter (23) to ensure its supply of electricity; and
- a lighting device (1), intended to be arranged outside said human or animal body, to perform transcutaneous lighting of said photovoltaic converters;

in which system:

said implantable electronic or electrical device comprises:

- an instrument (42) for measuring the electrical power generated by said photovoltaic converter; and
- a transmitter (42), to transmit a signal representative of said electrical power; and wherein said lighting device comprises:
- a receiver (41), for receiving said signal representative of the electrical power generated by said photovoltaic converter; and

**characterized in that** the lighting device further comprises:

- a plurality of light sources able to be lit or extinguished individually; and
- a controller (41) for calculating, from said signal, an energy transfer efficiency between said lighting device and said photovoltaic converter, and to individually light or extinguish the light sources so as to maximize said efficiency or ensure that it is above a predetermined threshold.

2. The system according to claim 1, wherein said controller is also configured or programmed to adjust the light power emitted by each lit light source so as to maximize said energy transfer efficiency or ensure that it is above a predetermined threshold.

3. The system according to one of the preceding claims, wherein said transmitter (42) is formed by said photovoltaic converter.

4. The system according to one of claims 1 or 2, wherein said transmitter (42) comprises means for modulating an incident light radiation on said implantable electronic or electrical device and reorienting it toward said lighting device.

5. The system according to one of the preceding claims, wherein the surface on which said light sources are actually arranged has an area larger than that of the photosensitive surface of said photovoltaic converter.

6. The system according to one of the preceding claims, wherein said light sources have a multidirectional light transmission.

7. The system according to one of the preceding claims, wherein said lighting device comprises a transparent film (61) covering said light sources, the optical index of said film being chosen so as to perform an index adaptation with the skin of said human or animal body.

8. The system according to claim 7, wherein said transparent film is covered by a layer of transparent gel, the optical index of said gel being chosen so as to perform an index adaptation with the skin of said human or animal body.

9. The system according to one of the preceding claims, wherein said lighting device comprises a flexible and conformable support (11).

10. The system according to claim 9, wherein the face of said flexible and conformable support (11) bearing said light sources is covered with a layer of transparent glue allowing it to be fastened to the skin of said human or animal body, the optical index of said layer of glue being chosen so as to perform an index adaptation with said skin.

11. The system according to one of the preceding claims, wherein said support is transparent or semi-transparent over at least part of its surface.

12. The system according to claim 11, wherein said or each said light source is semi-transparent.

13. The system according to one of the preceding claims, wherein said lighting device is suitable for emitting light radiation in the spectral range from 300 to 1900 nm and preferably in the spectral range from 750 to 1200 nm.

14. The system according to one of the preceding claims, wherein said implantable electronic or electrical device comprises a storage element (25) for the electricity generated by said photovoltaic converter.

15. The system according to one of the preceding claims, wherein the lighting device comprises a modulator of the light intensity emitted by said light sources, to send information to said implantable electronic or electrical device, and said implantable electronic or electrical device comprises a receiver to read the variations in the current or voltage generated by said photovoltaic converter and extract that information therefrom.

12

11

1

Fig. 1

1

11

12

21

2

23

25

Fig. 2

EP 2 858 717 B1

Fig. 3

Fig. 4

12

53

51

52

21

Fig. 5

21

61

62

11

63

12

Fig 6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6400991 B
- US 8082041 B
- US 6091015 A
- US 20060085051 A

**Littérature non-brevet citée dans la description**

- **K. MURAKAWA et al.** A wireless near-infrared energy system for medical implants. *IEEE Engineering Medecine Biology,* 1999, vol. 18, 70